(19) Europäisches Patentamt
European Patent Office
Office européen des brevets

(11) Numéro de publication: **0 346 207 B1**

(12) **FASCICULE DE BREVET EUROPEEN**

(45) Date de publication de fascicule du brevet: **02.03.94**

(51) Int. Cl.[5]: **C07D 471/04**, A61K 31/44, //(C07D471/04,221:00,221:00)

(21) Numéro de dépôt: **89401547.8**

(22) Date de dépôt: **05.06.89**

(54) **Dérivés d'amino-4 carboxy-3 naphtyridines, leur préparation et compositions pharmaceutiques qui les contiennent.**

(30) Priorité: **09.06.88 FR 8807723**

(43) Date de publication de la demande:
**13.12.89 Bulletin 89/50**

(45) Mention de la délivrance du brevet:
**02.03.94 Bulletin 94/09**

(84) Etats contractants désignés:
**AT BE CH DE ES FR GB GR IT LI LU NL SE**

(56) Documents cités:
**EP-A- 0 018 735**
**EP-A- 0 205 362**

**CHEMICAL ABSTRACTS, vol. 97, no. 19, 8 novembre 1982, page 706, résumé no. 162862n, Columbus, Ohio, US; R.M. TITKOVA et al.: "Synthesis of 4-substituted 3-carbethoxy[carboxy]-1,5-naphthyridines, their properties and biological activity"**

(73) Titulaire: **ELF SANOFI**
**32-34, rue Marbeuf**
**F-75008 Paris(FR)**

(72) Inventeur: **Mendes, Etienne**
**2, Place Jeanne d'Arc**
**F31000 Toulouse(FR)**
Inventeur: **Vernieres, Jean-Claude**
**Rue Sabatié Garat**
**F-31600 Muret(FR)**
Inventeur: **Simiand, Jacques, Edouard**
**136 Chemin de Lacombe**
**F-31600 Muret(FR)**
Inventeur: **Keane, Peter Eugène**
**8 rue Marcel Doret**
**Roquettes F-31120 Portet-sur-Garonne(FR)**

(74) Mandataire: **Polus, Camille et al**
**c/o Cabinet Lavoix**
**2, Place d'Estienne d'Orves**
**F-75441 Paris Cedex 09 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne des dérivés d'amino-4 carboxy-3 naphtyridines, leur procédé de préparation et les médicaments qui les comprennent.

L'invention a pour objet les composés de formule I

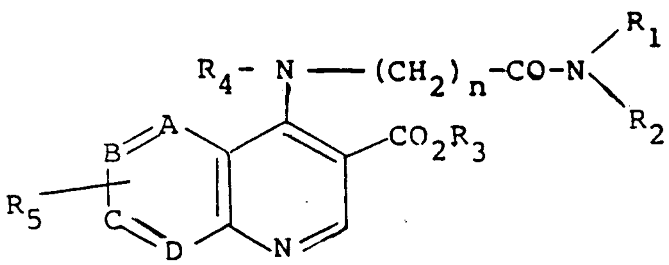

I

dans laquelle

$R_1$ et $R_2$, indépendamment l'un de l'autre, représentent l'atome d'hydrogène, un groupe alkyle en $C_1$ à $C_6$, un groupe phényle ou benzyle, ou $R_1$ et $R_2$ peuvent former avec l'atome d'azote auquel ils sont rattachés un hétérocycle saturé en $C_4$ à $C_8$,

$R_3$ et $R_4$, indépendamment l'un et l'autre, représentent l'atome d'hydrogène ou un groupe alkyle en $C_1$ à $C_6$,

$R_5$ représente un atome d'hydrogène ou d'halogène, ou un groupe alkyle en $C_1$ à $C_4$, alkoxy en $C_1$ à $C_4$, nitro ou trifluorométhyle,

n est 1,2 ou 3, et

l'un des symboles A, B, C ou D représente N et les autres représentent CH,

ainsi que les -N oxyde des azotes hétérocycliques et leurs sels d'addition avec des acides pharmaceutiquement acceptables, ou avec des bases pharmaceutiquement acceptables lorsque $R_3$ représente l'hydrogène.

Les groupes alkyles peuvent être linéaires, ramifiés ou cycliques.

Les groupes phényle ou benzyle peuvent être éventuellement substitués par les groupes alkyles en $C_1$ à $C_6$, alkoxy en $C_1$ à $C_6$, trifluorométhyle ou nitro, ou par les atomes d'halogène.

Les sels d'addition avec les acides peuvent être formés avec les acides minéraux comme les acides halogénohydriques, nitrique, sulfurique ou phosphorique ou avec les acides organiques comme les mono ou diacides carboxyliques, tels que les acides acétique, formique, succinique, tartrique, oxalique, aspartique, ou avec les acides sulfoniques, tels que l'acide méthane sulfonique ou benzène sulfonique.

Les sels avec les bases peuvent être des sels alcalins ou alcalino-terreux ou des sels avec des amines tel que la lysine, la pipérazine ou l'éthanolamine.

Parmi les produits préférés objets de l'invention, on peut citer les dérivés de naphtyridine-1,5 dans lesquels $R_3$ est méthyle ou éthyle, $R_4$ est l'hydrogène et n est 1, c'est à dire les dérivés de glycinamide, et plus particulièrement ceux pour lesquels $R_1$ et $R_2$ sont des groupes alkyles en $C_3$ à $C_5$ ou $R_1$ est un groupe alkyle en $C_1$ ou $C_2$ et $R_2$ est un groupe phényle ainsi que leurs sels.

L'invention concerne en outre un procédé de préparation des composés de formule I qui consiste à faire réagir un composé de formule II

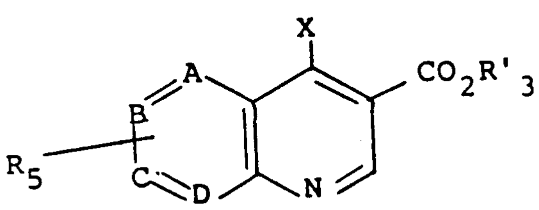

II

avec une amine de formule

$R_4NH(CH_2)_n\ CONR_1R_2$ III

dans lesquelles $R_1$,$R_2$,$R_4$,$R_5$,n,A,B,C,D, ont les mêmes significations que dans la formule I, $R'_3$ représente un groupe alkyle, et X représente un atome d'halogène ou un groupe sulfonate aliphatique ou aromatique.

La substitution de l'amine III peut être effectuée dans des conditions classiques, à une température comprise entre 60 ° C et 150 ° C, de préférence en présence d'une amine tertiaire ou d'une base minérale pour fixer l'acide HX formé, dans un solvant tel qu'un hydrocarbure aromatique, un alcool, ou un solvant aprotique polaire. On peut introduire (III) dans le milieu réactionnel sous forme de sel, avec une quantité équivalente de base pour libérer l'amine in situ.

On obtient les composés de formule I où $R_3$ = H en hydrolysant les esters correspondants, en milieu acide ou basique.

Les sels d'amines sont obtenus de façon classique, par action d'au moins un équivalent de l'acide sur les amines de formule I.

Certains composés de formule II sont connus; notamment, le dérivé de la naphtyridine-1,5 de formule

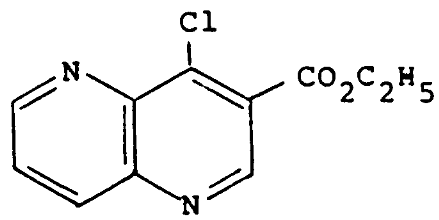

est décrit dans Chem. Abstr. 97 162862n; il est préparé par action de $POCl_3$ sur l'éthoxycarbonyl-3 hydroxy-4 naphtyridine-1,5 correspondante.

En général, les composés II pourront être préparés par l'intermédiaire des dérivés hydroxylés correspondants à partir des aminopyridines selon le schéma réactionnel :

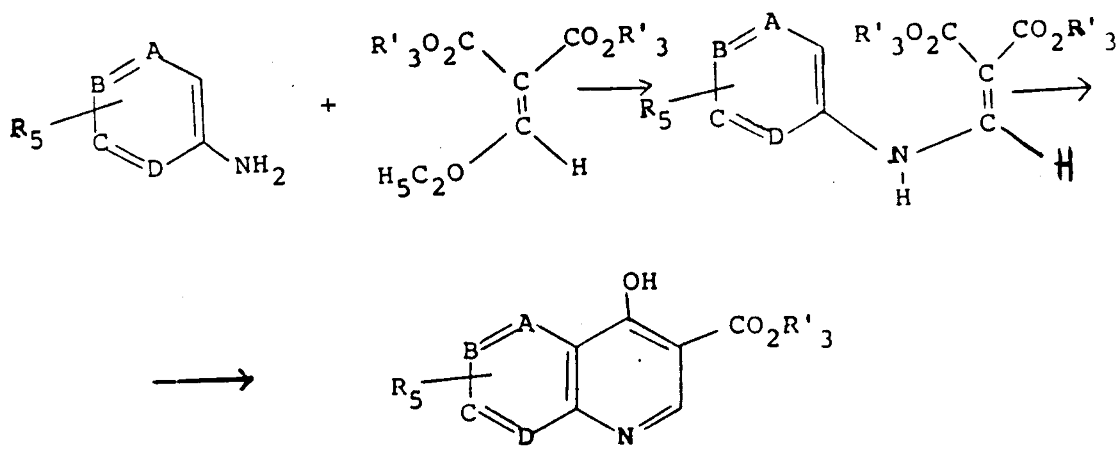

dans ces formules $R'_3$, $R_5$,A,B,C,D sont comme dans la formule II.

Les N-oxydes de ces composés ou des dérivés chlorés correspondants sont préparés par action d'un peracide selon une méthode connue.

Ces méthodes de préparation sont décrites dans Heterocyclic Compounds 7 p. 199-236 (1961) publié par John Wiley et dans Comprehensive Heterocyclic Chemistry p. 582-625 (1984) publié par Pergamon Press.

Certains composés de formule III sont connus; . ils peuvent autrement être préparés par des procédés décrits pour des produits homologues comme celui de R.D. Haworth et coll. dans J. Chem. Soc. p. 2972-2980 (1952) qui prépare des composés de formule III dans laquelle $R_4$ = H par l'intermédiaire du phtalimide :

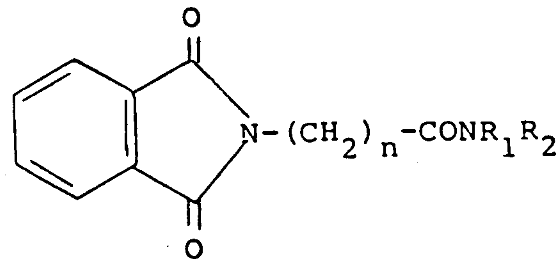

ou celui de G.B. BETTOLO et J.F. CAVALLA dans Gazz. Chim. Ital. p. 896-907 (1954) qui fait réagir l'amine $R_4NH_2$ sur le dérivé halogéné Cl-$(CH_2)$-$CONR_1R_2$ obtenu par action de $NHR_1R_2$ sur $ClCH_2COCl$ (dans ces formule $R_1$, $R_2$ et $R_4$ sont comme dans la formule I).

Les composés de formule I et leurs sels ont, in vitro et in vivo, une nette affinité pour les récepteurs centraux et périphériques des benzodiazépines et un autre objet de l'invention est constitué par les compositions pharmaceutiques qui contiennent comme principe actif au moins l'un des composés de l'invention.

On connaît déjà des naphtyridines actives sur les récepteurs centraux des benzodiazépines, telles que les naphtyridines-1,8 décrites dans EP-A-0 234 971, mais les substitutions portées par l'hétérocycle décrites dans ce document antérieur sont très différentes de celle des produits de formule I tandis que d'autres dérivés de structure plus proche, tels que les acides (amino-4 naphtyridine-1,5)-yl-3carboxyliques cités dans Chem. Abstr. 97 162862n, n'ont qu'une activité antimicrobienne, donc de nature très différente. Des quinoléines substituées de façon analogue et ayant une affinité pour les récepteurs des benzodiazépines sont également connues par EP-A-0 205 362.

Ces médicaments peuvent se présenter sous forme de compositions pharmaceutiques administrables par voie orale, rectale ou parentérale, dans lesquelles les composés de formule I et leurs sels sont associés aux excipients habituellement utilisés pour la préparation de comprimés, gélules, granulés, sirops, suppositoires ou solutés injectables.

On sait que les agonistes des récepteurs centraux des benzodiazépines ont selon leur structure, à intensité variable, des activités anxiolytiques, anticonvulsivantes, sédatives et hypnotiques, et que les ligands des récepteurs périphériques peuvent avoir quant à eux des activités anxiolytiques, vasodilatatrices ou immunomodulatrices. Les composés de l'invention seront utiles pour le traitement des états pathologiques correspondants, à la dose journalière de 5 mg à 300 mg, administrable en 1 à 3 prises, en fonction de la structure du composé, de l'âge du malade et de la gravité de son état.

Les composés de l'invention peuvent aussi être utilisés comme réactifs biochimiques.

Les exemples suivants illustrent l'invention. Les résultats d'analyse élémentaire des composés préparés répondent aux normes habituelles; les points de fusion mentionnés sont des points de fusion instantanés. Les déplacements chimiques en résonance magnétique nucléaire ont été déterminés par rapport à $Si(CH_3)_4$, comme étalon interne; q signifie quadruplet, t triplet, m massif, s singulet, d doublet et (xH) que la bande correspond à x protons.

Préparation de composés de formule II

1) chloro-4 naphtyridine-1,5-yl-3 carboxylate d'éthyle (formule II : $R'_3 = C_2H_5$, $R_5$ = H, A = N, B = C = D = CH, X = Cl)

On introduit, goutte à goutte, 0,64ml de $POCl_3$ dans une solution de 1,27g d'hydroxy-4 naphtyridine-1,5 yl-3 carboxylate d'éthyle, préparé en appliquant la méthode mentionnée dans Chem. Abstr. 97 162862n, et de 0,98 ml de triéthylamine dans 25 ml de tétrahydrofuranne, maintenu à sa température de reflux: on élimine le solvant et $POCl_3$ en excès par distillation sous pression réduite; le résidu est traité par l'eau glacée et le milieu est neutralisé par addition de $NH_4OH$ concentré avant l'extraction de la phase aqueuse par l'éther éthylique.

On obtient, après élimination du solvant organique, un solide qui est purifié par chromatographie sur une colonne de silice, en éluant par un mélange de cyclohexane et d'acétate d'éthyle (80/20) - Le produit pur fond à 71 °C.

2) dichloro-4,6 naphtyridine-1,5-yl-3 carboxylate d'éthyle (II : X = Cl, R'$_3$ = $C_2H_5$, $R_5$ = 6-Cl, A = N, B = C = D = CH)

a) préparation de la naphtyridine

Le mélange de 10,2g d'amino-5 chloro-2 pyridine et 16 ml d'éthoxyméthylènemalonate d'éthyle est chauffé à 140-150 ° C. L'éthanol formé est distillé au fur et à mesure de sa formation. Après purification par chromatographie sur une colonne de silice en éluant par un mélange de cyclohexane et d'acétate d'éthyle (8/2) on recueille le N-(chloro-2 pyridyl-5) aminométhylènemalonate d'éthyle. F = 126 ° C (cyclohexane) Rdt : 85%.

17g du composé précédent sont introduits sur du Dowtherm A (préparé à partir de 115,7 ml de diphényléther et 45g de biphényle) à 240 ° C. Cette solution est ensuite portée à 245 ° C et cette température maintenue pendant 45 mn. L'éthanol formé est distillé au fur et à mesure. Après refroidissement à température ambiante le précipité filtré est lavé à l'éther de pétrole. On obtient dans ces conditions un mélange de (chloro-6 hydroxy-4 naphtyridine-1,5)-yl-3 carboxylate d'éthyle et de (chloro-6 hydroxy-4 naphtyridine-1,7) yl-3 carboxylate d'éthyle à raison d'environ 95%, en poids, du premier composé.

La séparation des deux isomères n'a été effectuée qu'au stade du dérivé II.

b) Chloration

On introduit dans 100 ml de $POCl_3$, 12g du mélange d'isomères précédents et maintient 0,5heure à la température de reflux avant d'éliminer l'excès de $POCl_3$ par distillation sous pression réduite.

Le résidu est ensuite traité à l'eau glacée, neutralisée à une température comprise entre 5 et 10 ° C par addition d'une solution de NaOH aqueux; la phase aqueuse est alors extraite deux fois par 150 ml de dichlorométhane et les phases organiques sont concentrées après séchage sur Mg $SO_4$.

Les composés de formule II où X = Cl et A = N, B = C = D = CH (naphtyridine-1,5) et C = N, A = B = D = CH (naphtyridine-1,7) sont séparés par chromatographie sur colonne de silice en éluant avec un mé-lange de cyclohexane et d'acétate d'éthyle 95/5.

Le dérivé 1,5 fond à 114 ° C après recristallisation dans le cyclohexane 1H RMN (60 MHZ, $CDCl_3$) : 1,10-1,30 (t,3H) 4,10-4,60 (q,2H) 7,40-7,50 (d,1H) 8,05-8,30 (d,1H) 9,15 (s,1H) 9,0 (s,1H).

Le dérivé 1,7 a un spectre RMN différent, dans les mêmes conditions : 1,40-1,75 (t,3H) 4,40-4,80 (q,2H) 8,20 (s, 1H) 9,15 (s, 1H), 9,40 (s, 1H).

3) (dichloro-4,7 naphtyridine-1,8)yl-3 carboxylate d'éthyle

On porte 1 heure au reflux un mélange de 36 ml de $POCl_3$ et 10g de (chloro-7 hydroxy-4 naphtyridine-1,8)yl-3carboxylate d'éthyle préparé selon le procédé décrit ci-dessus en 2-a/ mais à partir d'amino-6 chloro-2 pyridine. Le produit fond à 134-136 ° C après recristallisation dans le cyclohexane.

4) (chloro-4 naphtyridine-1,6)yl-3 carboxylate d'éthyle (II : R'$_3$ = $C_2H_5$, $R_5$ = H, A = C = D = CH, B = N, X = Cl)

Ce produit est préparé en appliquant le mode opératoire décrit par M.J. Weiss dans Heterocyclic compounds (1961) 7, p. 216, Elderfield, à partir d'(hydroxy-4 naphtyridine-1,6)yl-3 carboxylate d'éthyle. Il fond à 72 ° C.

5) 1-oxyde de (dichloro-4,6 naphtyridine 1,5)yl-3 carboxylate d'éthyle (II : R'$_3$ = $C_2H_5$, $R_5$ = 6-Cl, A = N, B = C = D = CH, X = Cl)

On agite 96 heures à température ambiante 10g de (dichloro-4,6 naphtyridine-1,5)yl-3 carboxylate d'éthyle et 13,8g d'acide métachloroperbenzoïque dans 200ml d'acide acétique. Le solvant est alors distillé sous pression réduite et le résidu cristallisé dans l'hexane froid; Le précipité est dissous dans le dichlorométhane et la solution est lavée par une solution aqueuse de bicarbonate de sodium puis à l'eau.

Le solvant est évaporé et le résidu recristallisé dans l'éthanol. Il fond vers 140-150 ° C.

EXEMPLE 1

[chloro-6 (N,N-dipropylcarbamoylméthylamino)-4 naphtyridine-1,5] yl-3 carboxylate d'éthyle (référence : SR 25966).

Formule I : $R_1 = R_2 = C_3H_7$, $R_3 = C_2H_5$, $R_4 = H$, $R_5 = 6\text{-Cl}$, A = N, B = C = D = CH, n = 1;

On maintient à la température de reflux, durant 1 heure, 3,9g de (dichloro-4,6 naphtyridine-1,5)yl-3 carboxylate d'éthyle, préparé comme précédemment indiqué, avec 2,7g de chlorhydrate de N,N-dipropyl amino-2 acétamide et 4,5 ml de triéthylamine dans 30 ml d'éthanol. Après élimination du solvant par distillation sous pression réduite, le résidu est dissous dans 50 ml de dichlorométhane et la phase organique est lavée à l'eau, et séchée avant élimination du solvant.

Le résidu peut être purifié par chromatographie sur une colonne de silice, en éluant par un mé-lange de cyclohexane et d'acétate d'éthyle avant recristallisation dans le cyclohexane. F = 126 °C (Rdt 72%-.

$^1$H-RMN (80 MHZ, CDCl3) $\delta$ (ppm) : 0,80-1,20 (q,6H) 1,25-1,50 (t,3H) 1,50-1,85 (m,4H) 3,10-3,50 (q,4H) 4,20-4,50 (q,2H) 4,80-5,00 (d,2H) 7,18-7,36 (d,1H) 7,90-8,10 (1H) 9,10 (s, 1H) 10,30-10,55 (m, 1H échangeable).

EXEMPLES 2 à 27

Les naphtyridines-1,5, de formule I dans laquelle n = 1 ou 2, préparées en appliquant le procédé décrit à l'exemple 1, figurent dans le tableau I suivant.

L'acide de l'exemple 22 a été préparé par action sur 2,5g de l'ester éthylique correspondant d'une solution de 60 ml de NaOH 0,5N pendant 2 heures au reflux.

Le composé de l'exemple 27 est le N-oxyde du composé de l'exemple 26.

TABLEAU 1

| EX N° | SR | n | $R_1$ | $R_2$ | $R_3$ | $R_4$ | $R_5$ | F°C | SOLVANT DE RECRISTALLIS. |
|---|---|---|---|---|---|---|---|---|---|
| 2 | 26040 | 1 | $C_2H_5$ | $C_2H_5$ | $C_2H_5$ | H | 6-Cl | 148 | Cyclohexane |
| 3 | 26111 | 1 | $C_4H_9$ | $C_4H_9$ | $C_2H_5$ | H | 6-Cl | 109 | Cyclohexane |
| 4 | 26292 | 1 | $C_5H_{11}$ | $C_5H_{11}$ | $C_2H_5$ | H | 6-Cl | 118 | Cyclohexane |
| 5 | 26056 | 1 | $CH_3$ | H | $C_2H_5$ | H | 6-Cl | 213 | $CH_3COOC_2H_5$ |
| 6 | 26023 | 1 | $C_3H_7$ | H | $C_2H_5$ | H | 6-Cl | 218 | $CH_3COOC_2H_5$ |
| 7 | 26105 | 1 | i-$C_3H_7$ | i-$C_3H_7$ | $C_2H_5$ | H | 6-Cl | 177 | Cyclohexane |
| 8 | 26155 | 1 | i-$C_4H_9$ | i-$C_4H_9$ | $C_2H_5$ | H | 6-Cl | 141 | Cyclohexane |
| 9 | 26057 | 1 | $-(CH_2)_6-$ | | $C_2H_5$ | H | 6-Cl | 171 | Cyclohexane |
| 10 | 26297 | 1 | $CH_3$ | $C_4H_9$ | $C_2H_5$ | H | 6-Cl | 115 | Cyclohexane |
| 11 | 26508 | 1 | $CH_3$ | sec-$C_4H_9$ | $C_2H_5$ | H | 6-Cl | 129 | Cyclohexane |
| 12 | 26081 | 1 | $CH_3$ | $C_6H_5$ | $C_2H_5$ | H | 6-Cl | 175 | Benzène |
| 13 | 26242 | 1 | $CH_3$ | (Cl-4)$C_6H_4$ | $C_2H_5$ | H | 6-Cl | 240 | $CH_3COOC_2H_5$ |
| 14 | 25998 | 1 | $C_3H_7$ | $C_3H_7$ | $C_2H_5$ | $CH_3$ | 6-Cl | 108 | Ether pétrole |
| 15 | 26130 | 1 | $C_3H_7$ | $C_3H_7$ | $CH_3$ | H | 6-Cl | 151 | Cyclohexane |
| 16 | 26216 | 1 | $C_3H_7$ | $C_3H_7$ | $C_2H_5$ | $CH_3$ | 6-$CH_3$ | 136 (maléate) | $CH_3COOC_2H_5$ |
| 17 | 26177 | 1 | $C_2H_5$ | $C_2H_5$ | $C_2H_5$ | H | 6-$CH_3$ | 118 | Cyclohexane |
| 18 | 26212 | 1 | $C_3H_7$ | $C_3H_7$ | $C_2H_5$ | H | 6-$CH_3$ | 152 | Cyclohexane |
| 19 | 26035 | 1 | $C_3H_7$ | $C_3H_7$ | $C_2H_5$ | H | 6-$OCH_3$ | 139 | Cyclohexane |
| 20 | 26099 | 1 | $C_3H_7$ | $C_3H_7$ | $C_2H_5$ | $CH_3$ | 6-$OCH_3$ | 117 (maléate) | |
| 21 | 26041 | 1 | $C_3H_7$ | $C_3H_7$ | $C_2H_5$ | H | H | 152 | Cyclohexane |
| 22 | 26236 | 1 | $C_3H_7$ | $C_3H_7$ | H | H | 6-Cl | 260 | $C_2H_5OH$ |
| 23 | 26675 | 1 | $CH_3$ | (Cl-2)$C_6H_4$ | $C_2H_5$ | H | 6-Cl | 186 | Cyclohexane |
| 24 | 26973 | 1 | $CH_3$ | (Cl-4)$C_6H_4$ | $C_2H_5$ | H | H | 178 | Cyclohexane |
| 25 | 27250 | 1 | $-(CH_2)_5-$ | | $C_2H_5$ | H | 6-Cl | 168 | Cyclohexane |
| 26 | 26762 | 2 | $C_3H_7$ | $C_3H_7$ | $C_2H_5$ | H | 6-Cl | 98 | Cyclohexane |
| 27* | 26834 | 2 | $C_3H_7$ | $C_3H_7$ | $C_2H_5$ | H | 6-Cl | 120 | Cyclohexane |

* N-oxyde

EXEMPLE 28 :

[chloro-6 (N,N-dipropylcarbamoylméthylamino)-4 naphtyridine-1,7]-yl-3 carboxylate d'éthyle. (SR 26224) : (Formule I : $R_1 = R_2 = C_3H_7$; $R_3 = C_2H_5$; $R_4 = H$; $R_5$ =6-Cl; A = B = D = CH; C = N; n = 1).

Préparation selon le procédé de l'exemple 1 à partir de 0,83g de (dichloro-4,6 naphtyridine-1,7)-yl-3 carboxylate d'éthyle, de 0,65g de chlorhydrate de N,N-dipropyl amino-2 acétamide, de 0,94 ml de triéthylamine et 20 ml d'éthanol absolu. Le résidu après purification sur colonne de silice (éluant : toluène et éthanol; 99-1) est recristallisé dans le cyclohexane. F = 150 °C. (Rdt :85%).

$^1$H-RMN (60 MHz CDCl3) δ : 0,80-1,10 (t,6H) 1,20-1,95 (m,7H) 2,10-2,50 (q,4H) 4,30-4,65 (m,4H) 7,95 (s,1H) 9,05 (s,1H), 10,10-10,30 (m,1H).

EXEMPLE 29 :

[(N-méthyl N-(chloro-4 phényl) carbamoylméthylamino)-4 naphtyridine-1,7]-yl-3 carboxylate d'éthyle (SR 26773) formule I : $R_1 = CH_3$; $R_2$ = Cl-4$C_6H_4$; $R_3 = C_2H_5$; $R_4 = R_5$ = H; A = B = D = CH; C = N; n = 1.

Préparation selon le procédé de l'exemple 1 à partir de 0,69 g de (chloro-4 naphtyridine-1,7)-yl-3 carboxylate d'éthyle, décrit par J.C. Murray et C.R. Hauser dans J. Org. Chem. p. 2008-2014 (1954), de 0,66g de chlorhydrate de N-méthyl N-chloro-4 phénylamino-2 acétamide dans 10 ml d'éthanol en présence de 0,8 ml de triéthylamine. Le produit final est isolé par chromatographie sur colonne de silice en éluant avec un mélange de toluène et d'éthanol (95/5-v/v), et recristallisé dans le toluène. F = 242 °C (Rdt : 32%).

$^1$HRMN (80 MHz, $CDCl_3$) δ : 1,2-1,5 (t, 3H); 3,3 (s,3H); 4,1-4,6 (m,4H); 7,1-7,6 (m,5H); 8,3-8,5 (d,1H); 9,1 (s,1H); 9,2 (s,1H); 9,8-10,1 (m, 1H)

EXEMPLES 30 à 32 : dérivés de naphtyridine-1,8 préparés comme précédemment; on obtient :

Exemple 30 : (SR 26305)

formule I : $R_1 = R_2 = C_3H_7$, $R_3 = C_2H_5$, $R_4$ = H, $R_5$ = 7-Cl, A = B = C = CH, D = N, n = 1.

On maintient 2 heures au reflux une solution de 1,28g de chlorhydrate d'amino-2 N,N-dipropylacétamide, 1,63g de (dichloro-4,7 naphtyridine-1,8)-yl-3 carboxylate d'éthyle, 1,85ml de triéthylamine dans 30ml d'éthanol.

Les produits volatils sont éliminés sous pression réduite et le produit final extrait du résidu par le cyclohexane dans un Soxhlet, avec 75% de rendment. F = 151 °C.

$^1$H-RMN (60MHz, $CDCl_3$) δ : 0,70-1,10 (t,6H) 1,30-1,90 (m,7H) 3,00-3,60 (q,4H) 4,20-4,70 (m,4H) 7,00-7,20 (d,1H) 8,30-8,50 (d,1H) 9,0 (s,1H) 10,5 (m,1H).

Exemple 31 : (SR 26285)

formule I : $R_1 = R_2 = C_3H_7$, $R_3 = C_2H_5$, $R_4$ = H, $R_5$ = 7-$CH_3$, A = B = C = CH, D = N, n = 1.

F = 162 °C (recristallisation dans l'acétate d'éthyle)

$^1$H-RMN (60 MHz, $CDCl_3$) δ : 0,70-1,05 (m,6H) 1,30-1,90 (m,7H) 2,60 (s,3H) 3,0-3,55 (q,4H) 4,20-4,60 (m,4H) 6,95-7,10 (d,1H) 8,30-8,50 (d,1H) 9,10 (s, 1H) 10,10-10,40 (m,1H).

Exemple 32 : (SR 26323)

Formule I : $R_1 = CH_3$, $R_2$ = (Cl-4)$C_6H_4$, $R_3 = C_2H_5$, $R_4$ = H, $R_5$ = 7-Cl, A = B = C = CH, D = N, n = 1.

F = 174 °C (recristallisation dans l'acétonitrile).

Exemple 33 :

[(N-méthyl N-(chloro-4 phényl)carbamoylméthylamino)-4 naphtyridine-1,6]-yl-3 carboxylate d'éthyle (SR 26625) (Formule I : $R_1 = CH_3$ ; $R_2$ = (Cl-4)$C_6H_4$; $R_3 = C_2H_5$; $R_4 = R_5$ = H; A = C = D = CH; B = N; n = 1).

F = 232 ° C après recristallisation dans l'éthanol.

$^1$HRMN (80 MHz,$CDCl_3$) δ : 1,20-1,60 (t,3H); 3,4(s,3H); 4,1-4,6 (m,4H); 7,1-7,7 (m, 5H); 8,4-8,6 (d, 1H); 9-9,2 (d, 2H); 10,4-10,7 (m, 1H).

Exemple 34 :

[(N,N-dipropylcarbamoylméthylamino)-4 naphtyridine-1,6]-yl-3 carboxylate d'éthyle (SR 25874) (formule I : $R_1$ = $R_2$ = $C_3H_7$; $R_3$ = $C_2H_5$; $R_4$ = $R_5$ = H; A = C = D = CH; B = N; n=1).

F = 145 ° après recristallisation dans le cyclohexane.

Exemple 35 : comprimés avec le produit de l'exemple 15.

Les comprimés sont préparés selon les techniques habituelles avec 30 mg de SR 26130 et comme excipients du lactose (80mg), de l'amidon de maïs (4mg), du stéarate de magnésium (1mg) et de la polyvinylpyrrolidone (2mg).

Exemple 36 : gélules avec le produit de l'exemple 1.

25mg de SR 25966 sont introduits dans des gélules de taille 1 compacté avec 10mg de cellulose, 30mg de lactose, 5mg de talc, 5mg de carboxyméthylamidon sodique et 1mg de stéarate de magnésium.

Les composés des exemples précédents sont peu toxiques : à titre indicatif, on peut préciser que la $DL_{50}$ chez la souris, est de 1800mg/kg pour le composé de l'exemple 1, administré par voie orale.

On a aussi déterminé leur affinité, in vitro, pour les récepteurs des benzodiazépines centraux et périphériques et, pour certains d'entre eux, leur activité anxiolytique, hypnotique et anticonvulsivante, in vivo.

a) Affinité pour les récepteurs centraux des banzodiazépines, in vitro :

On a appliqué une technique analogue à celle décrite par Braestrup et Squires dans Proc. Nat. Acad. Sc. USA 74 (9) p. 3805-3809 (1977), mais en incubant à 4 ° C, durant 90 mn, en présence de 1,5 mM de $^3H$-flunitrazepam.

Les résultats figurent dans le Tableau II

TABLEAU II

| EXEMPLE | CI50 (nM) | EXEMPLE | CI50 (nM) |
|---|---|---|---|
| 1 | 45 | 18 | 20 |
| 2 | 182 | 19 | 143 |
| 4 | 1520 | 20 | 3280 |
| 8 | 172 | 21 | 396 |
| 10 | 133 | 22 | 3,6 |
| 12 | 27 | 23 | 22 |
| 13 | 59 | 24 | 738 |
| 14 | 2750 | 26 | 190 |
| 15 | 18 | 27 | 84 |
| 16 | 674 | 33 | 858 |
| 17 | 186 | diazepam | 11 |
| | | Chlordia-zepoxide | 654 |

B) Affinité pour les récepteurs périphériques des benzodiazépines, in vitro :

On a déterminé la CI50, concentration du produit testé inhibant de 50% la fixation spécifique du PK 11195, ligand connu des récepteurs de type périphérique; la forme tritiée de ce composé, le N-méthyl-1 propyl) (chloro-2 phényl-1) isoquinolyl-3 carboxamide, est commercialisée par le CEA (France). La méthode appliquée est analogue à celle de J. Benavides et coll. décrite dans Brain Res. Bull. 60-77 (1984).

Les résultats obtenus figurent dans le tableau III qui suit :

TABLEAU III

| EXEMPLE | CI50 (nM) | EXEMPLE | CI50 (nM) |
|---|---|---|---|
| 1 | 26 | 20 | 276 |
| 2 | 270 | 21 | 58 |
| 4 | 18 | 22 | 223 |
| 8 | 18 | 23 | 0,26 |
| 10 | 55 | 24 | 2,6 |
| 12 | 9,2 | 26 | 35 |
| 13 | 0,53 | 27 | 282 |
| 14 | 213 | 28 | 622 |
| 15 | 12 | 29 | 62 |
| 16 | 393 | 32 | 10 |
| 17 | 114 | 33 | 107 |
| 18 | 33 | | |
| 19 | 98 | | |

c) Activité anticonvulsivante :

On a étudié dans quelle mesure les produits protègent les animaux contre les crises convulsives déclenchées par l'administration de pentétrazole selon la méthode décrite par Everett etRichards dans J. Pharm. Exp. Ther. p. 402-407 (1944).

les produits à tester sont administrés par voie orale à des lots de 10 souris, 30 minutes avant l'injection par voie sous-cutanée de 125mg/kg de pentétrazole. On note le nombre d'animaux ne présentant pas de crise tonique pendant les 30 minutes suivant l'administration de l'agent convulsivant et détermine la dose efficace 50 c'est-à-dire la dose pour laquelle 50% des animaux sont protégés.

Les résultats, ainsi que ceux obtenus avec des anticonvulsivants connus, figurent dans le tableau IV; les limites de confiance, à 95%, ont été calculées en appliquant la méthode de Finney.

TABLEAU IV

| EXEMPLE | $DE_{50}$ (mg/kg) (intervalle confiance) |
|---|---|
| 1 | 11 (7-16) |
| 19 | 22 (15-19) |
| 15 | 11 (19-43) |
| chlordiazépoxide | 7 |
| phénobarbital | 10 |
| méprobamate | 36 |

d) Activité anxiolytique :

La méthode de R.J. Stephens décrite dans Brit. J. Pharmacol. p. 145P (1973) a été appliquée.

On sait que les anxiolytiques s'opposent à l'inhibition de la prise de nourriture chez la souris placée dans un lieu inconnu devant une nourriture inconnue.

Le composé de l'exemple 19, administré par voie orale à la dose de 32mg/kg augmente de 130% la consommation de nourriture, tandis que, dans les mêmes conditions, le chlordiazépoxide à la dose de 16 mg/kg ne l'augmente que de 73%, le diazépam à la dose de 4mg/kg de 80% et le méprobamate à 60mg/kg de 95%.

e) Activité hypnotique, in vivo :

L'activité a été étudiée en appliquant la méthode de Janssen, décrite dans J. Med. Pharm. Chem. p. 281 (1959). On a ainsi déterminée que la DE50 du composé de l'exemple 1 était 8mg/kg par voie I.V., alors que celle du thiopental sodique est de 17 mg/kg.

**Revendications**

**Revendications pour les Etats contractants suivants : AT, BE, CH, DE, FR, GB, IT, LI, LU, NL, SE**

**1.** Composés de formule I

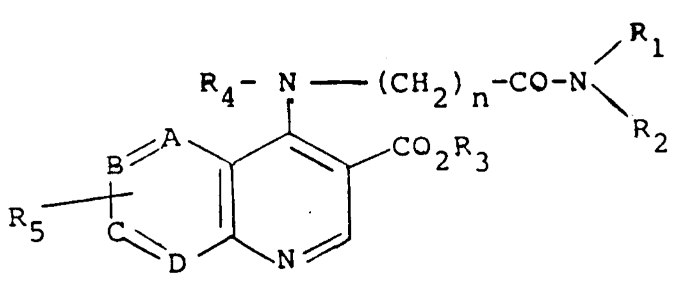

I

dans laquelle

$R_1$ et $R_2$, indépendamment l'un de l'autre, représentent l'atome d'hydrogène, un groupe alkyle en $C_1$ à $C_6$, un groupe phényle ou benzyle, ou $R_1$ et $R_2$ peuvent former avec l'atome d'azote auquel ils sont rattachés un hétérocycle saturé en $C_4$ à $C_8$,

$R_3$ et $R_4$, indépendamment l'un et l'autre, représentent l'atome d'hydrogène ou un groupe alkyle en $C_1$

à $C_6$,
$R_5$ représente un atome d'hydrogène ou d'halogène, ou un groupe alkyle en $C_1$ à $C_4$, alkoxy en $C_1$ à $C_4$, nitro ou trifluorométhyle, étant entendu que les groupes alkyles peuvent être linéaires. ramifiés ou cycliques, et que les groupes phényle ou benzyle peuvent être éventuellement substitués par les groupes alkyles en $C_1$ à $C_6$, alkoxy en $C_1$ à $C_6$, trifluorométhyle ou nitro, ou par les atomes d'halogène,
n est 1,2 ou 3, et
l'un des symboles A, B, C ou D représente N et les autres représentent CH,
ainsi que les N-oxydes des azotes hétérocycliques et leurs sels d'addition avec des acides et les bases pharmaceutiquement acceptables.

**2.** Composés selon la revendication 1, pour lesquels A de la formule I représente N et B,C,D représentent chacun CH.

**3.** Composés selon l'une des revendications 1 et 2 pour lesquels $R_4$ représente H et n est 1 dans la formule I.

**4.** Procédé de préparation de composés de formule I selon la revendication 1, caractérisé en ce qu'on fait réagir le dérivé de formule II

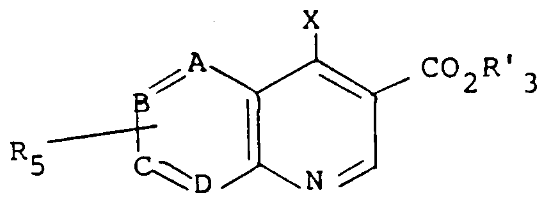

II

dans laquelle X représente un halogène ou un groupe sulfonyle, $R'_3$ représente un groupe alkyle, et $R_5$ et A,B,C,D ont les mêmes signification que dans la formule I,
avec une amine de formule III :

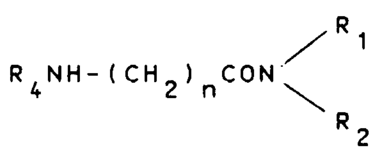

III

dans laquelle $R_1$, $R_2$, $R_4$ et n ont la même signification que dans la formule I, puis le cas échéant on hydrolyse les esters (I) ainsi obtenus en acides correspondants ($R_3$ = H).

**5.** Composition pharmaceutique comprenant comme principe actif un composé selon l'une des revendications 1 à 3.

**Revendications pour les Etats contractants suivants : ES, GR**

**1.** Procédé de préparation de composés de formule I

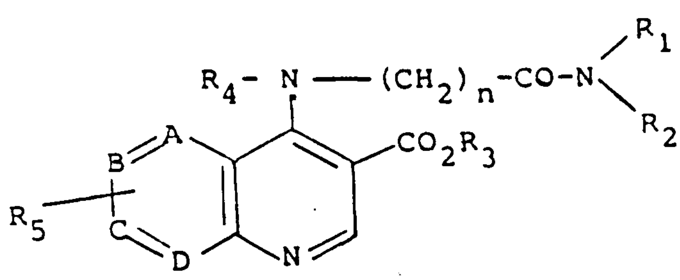

I

dans laquelle

$R_1$ et $R_2$, indépendamment l'un de l'autre, représentent l'atome d'hydrogène, un groupe alkyle en $C_1$ à $C_6$, un groupe phényle ou benzyle, ou $R_1$ et $R_2$ peuvent former avec l'atome d'azote auquel ils sont rattachés un hétérocycle saturé en $C_4$ à $C_8$,
$R_3$ et $R_4$, indépendamment l'un et l'autre, représentent l'atome d'hydrogène ou un groupe alkyle en $C_1$ à $C_6$,
$R_5$ représente un atome d'hydrogène ou d'halogène, ou un groupe alkyle en $C_1$ à $C_4$, alkoxy en $C_1$ à $C_4$, nitro ou trifluorométhyle,
étant entendu que les groupes alkyles peuvent être linéaires, ramifiés ou cycliques, et que les groupes phényle ou benzyle peuvent être éventuellement substitués par les groupes alkyles en $C_1$ à $C_6$, alkoxy en $C_1$ à $C_6$, trifluorométhyle ou nitro, ou par les atomes d'halogène,
n est 1,2 ou 3, et
l'un des symboles A, B, C ou D représente N et les autres représentent CH,
ainsi que les N-oxydes des azotes hétérocycliques et leurs sels d'addition avec des acides et les bases pharmaceutiquement acceptables, caractérisé en ce qu'on fait réagir le dérivé de formule II

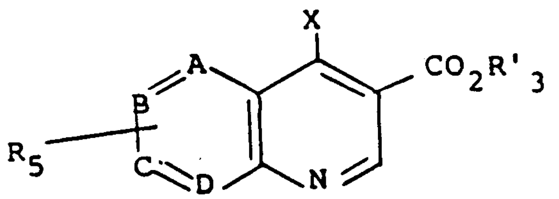

II

dans laquelle X représente un halogène ou un groupe sulfonyle, $R'_3$ représente un groupe alkyle et $R_5$ et A,B,C,D ont les mêmes signification que dans la formule I,
avec une amine de formule III :

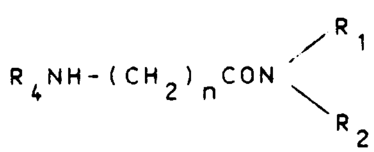

III

dans laquelle $R_1$,$R_2$,$R_4$ et n ont la même signification que dans la formule I, puis le cas échéant on hydrolyse les esters (I) ainsi obtenus en acides correspondants ($R_3$ = H).

**2.** Procédé selon la revendication 1, caractérisé en ce qu'on prépare les composés pour lesquels A de la formule I représente N et B,C,D représentent chacun CH.

**3.** Procédé selon l'une des revendications 1 et 2 caractérisé en ce que l'on prépare les composés pour lesquels $R_4$ représente H et n est 1 dans la formule I.

**4.** Procédé de préparation d'une composition pharmaceutique comprenant comme principe actif un composé obtenu selon l'une des revendications 1 à 3, caractérisé en ce que l'on associe ce composé aux excipients habituellement utilisés.

## Claims

**Claims for the following Contracting States : AT, BE, CH, DE, FR, GB, IT, LI, LU, NL, SE**

1. Compounds of formula I

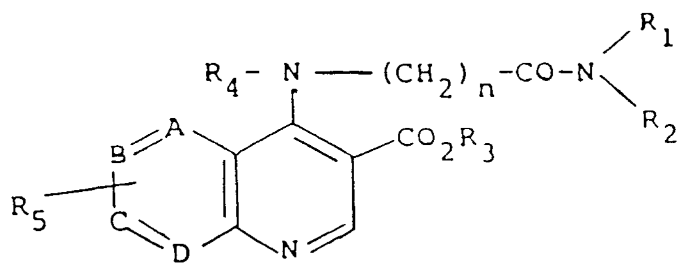

I

wherein

$R_1$ and $R_2$, independently of each other, represent a hydrogen atom, a $C_{1-6}$ alkyl, a phenyl or benzyl group, or $R_1$ and $R_2$ may, together with the nitrogen atom to which they are linked, form a saturated $C_{4-8}$ heterocyclic group,

$R_3$ and $R_4$, independently of each other, represent a hydrogen atom or a $C_{1-6}$ alkyl group,

$R_5$ represents a hydrogen or halogen atom or a $C_{1-4}$ alkyl, $C_{1-4}$ alkoxy, nitro or trifluoromethyl group,

given that the alkyl groups may be straight chained, branched or cyclic, and that the phenyl or benzyl groups may optionally be substituted by $C_{1-6}$ alkyl, $C_{1-6}$ alkoxy, trifluoromethyl or nitro groups or by halogen atoms,

n is 1, 2 or 3 and

one of the symbols A, B, C or D represents N and the others represent CH,

and the N-oxides of the heterocyclic nitrogens and the addition salts thereof with pharmaceutically acceptable acids and bases.

2. Compounds according to claim 1 wherein A in formula 1 denotes N and B, C, D each denote CH.

3. Compounds according to one of claims 1 and 2, wherein $R_4$ denotes H and n is 1 in formula I.

4. The process for preparing compounds of formula 1 according to claim 1, characterized in that the derivative of formula II

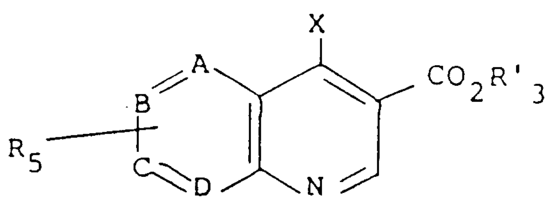

II

wherein X denotes a halogen or a sulphonyl group, $R'_3$ represents an alkyl group and $R_5$ and A, B, C, D have the same meanings as in formula I, is reacted with an amine of formula III

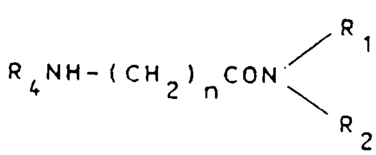

III

wherein $R_1$, $R_2$, $R_4$ and n have the same meanings as in formula I, then if required esters (I) thus obtained are hydrolyzed into the corresponding acids ($R_3 = H$).

5. Pharmaceutical composition comprising as active substance a compound according to one of claims 1-3.

**Claims for the following Contracting States : ES, GR**

1. Process for preparing compounds of formula I

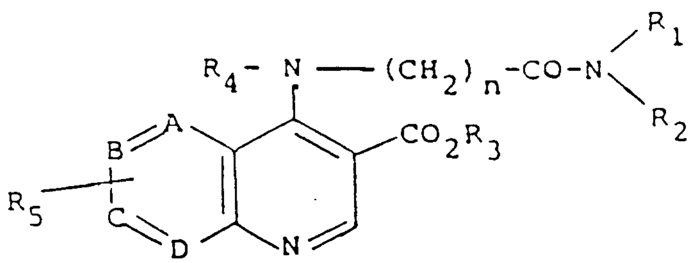

I

wherein

$R_1$ and $R_2$, independently of each other, represent a hydrogen atom, a $C_{1-6}$ alkyl, a phenyl or benzyl group, or $R_1$ and $R_2$ may, together with the nitrogen atom to which they are linked, form a saturated $C_{4-8}$ heterocyclic group,

$R_3$ and $R_4$, independently of each other, represent a hydrogen atom or a $C_{1-6}$ alkyl group,

$R_5$ represents a hydrogen or halogen atom or a $C_{1-4}$ alkyl, $C_{1-4}$ alkoxy, nitro or trifluoromethyl group,

given that the alkyl groups may be straight chained, branched or cyclic, and that the phenyl or benzyl groups may optionally be substituted by $C_{1-6}$ alkyl, $C_{1-6}$ alkoxy, trifluoromethyl or nitro groups or by halogen atoms,

n is 1, 2 or 3 and

one of the symbols A, B, C or D represents N and the others represent CH,

and the N-oxides of the heterocyclic nitrogens and the addition salts thereof with pharmaceutically acceptable acids and bases, characterised in that the derivative of formula II

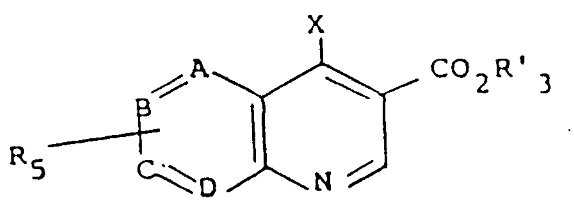

II

wherein X denotes a halogen or a sulphonyl group, R'$_3$ represents an alkyl group and $R_5$ and A, B, C, D have the same meanings as in formula I, is reacted with an amine of formula III

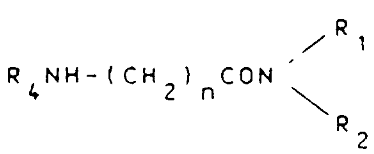

III

wherein $R_1$, $R_2$, $R_4$ and n have the same meanings as in formula I, then if required esters (I) thus obtained are hydrolysed into the corresponding acids ($R_3$ = H).

2. Process according to claim 1 characterised in that compounds are prepared wherein A in formula I denotes N and B, C, D each denote CH.

3. Process according to one of claims 1 and 2, characterised in that compounds are prepared wherein $R_4$ denotes H and n is 1 in formula I.

4. Process for preparing a pharmaceutical composition containing as active principle a compound obtained according to one of claims 1-3, characterised in that this compound is combined with the excipients conventionally used.

## Patentansprüche
## Patentansprüche für folgende Vertragsstaaten : AT, BE, CH, DE, FR, GB, IT, LI, LU, NL, SE

1. Verbindungen der Formel I

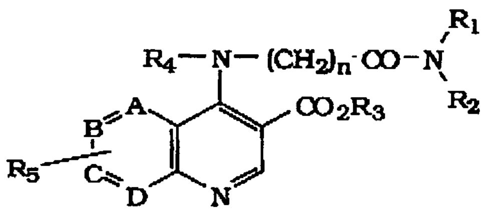

I

in der
$R_1$ und $R_2$ unabhängig voneinander Wasserstoffatome, $C_1$-$C_6$-Alkylgruppen, Phenylgruppen oder Benzylgruppen oder $R_1$ und $R_2$ gemeinsam mit dem Stickstoffatom, an das sie gebunden sind, einen gesättigten $C_4$-$C_8$-Heterocyclus,
$R_3$ und $R_4$ unabhängig voneinander Wasserstoffatome oder $C_1$-$C_6$-Alkylgruppen,
$R_5$ ein Wasserstoffatom, ein Halogenatom, eine $C_1$-$C_4$-Alkylgruppe, eine $C_1$-$C_4$-Alkoxygruppe, eine Nitrogruppe oder eine Trifluormethylgruppe,
mit der Maßgabe, daß die Alkylgruppen geradkettig, verzweigt oder cyclisch sein können und die Phenyl- oder Benzylgruppen gegebenenfalls durch $C_1$-$C_6$-Alkylgruppen, $C_1$-$C_6$-Alkoxygruppen, Trifluormethylgruppen, Nitrogruppen oder Halogenatome substituiert sein können,
n 1, 2 oder 3 und
eines der Symbole A, B, C oder D N und die anderen CH bedeuten,
sowie die N-Oxide der heterozyklischen Stickstoffatome und ihrer Additionssalze mit pharmazeutisch annehmbaren Säuren und Basen.

2. Verbindungen nach Anspruch 1, worin A der Formel I N und B, C und D jeweils CH bedeuten.

3. Verbindungen nach einem der Ansprüche 1 und 2, worin in der Formel I $R_4$ ein Wasserstoffatom und n 1 bedeuten.

4. Verfahren zur Herstellung der Verbindungen der Formel I gemäß Anspruch 1, **dadurch gekennzeichnet**, daß man ein Derivat der Formel II

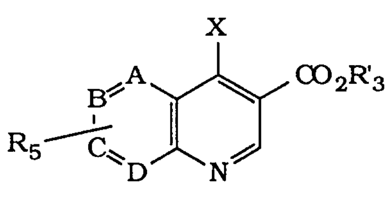

II

in der X ein Halogenatom oder eine Sulfonylgruppe und $R'_3$ eine Alkylgruppe bedeuten und $R_5$, A, B, C und D die bezüglich der Formel I angegebenen Bedeutungen besitzen,
mit einem Amin der Formel III:

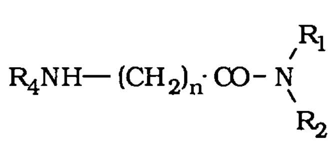

III

in der $R_1$, $R_2$, $R_4$ und n die bezüglich der Formel I angegebenen Bedeutungen besitzen, umsetzt und gegebenenfalls die in dieser Weise erhaltenen Ester (I) zu den entsprechenden Säuren hydrolysiert ($R_3$ = H).

**5.** Pharmazeutische Zubereitung enthaltend als Wirkstoff eine Verbindung nach einem der Ansprüche 1 bis 3.

**Patentansprüche für folgende Vertragsstaaten : ES, GR**

**1.** Verfahren zur Herstellung von Verbindungen der Formel I

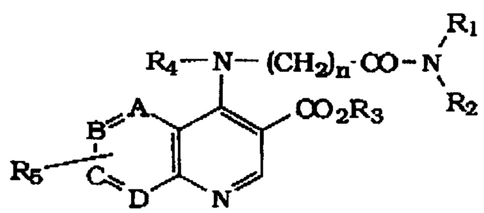

I

in der
$R_1$ und $R_2$ unabhängig voneinander Wasserstoffatome, $C_1$-$C_6$-Alkylgruppen, Phenylgruppen oder Benzylgruppen oder $R_1$ und $R_2$ gemeinsam mit dem Stickstoffatom, an das sie gebunden sind, einen gesättigten $C_4$-$C_8$-Heterocyclus,
$R_3$ und $R_4$ unabhängig voneinander Wasserstoffatome oder $C_1$-$C_6$-Alkylgruppen,
$R_5$ ein Wasserstoffatom, ein Halogenatom, eine $C_1$-$C_4$-Alkylgruppe, eine $C_1$-$C_4$-Alkoxygruppe, eine Nitrogruppe oder eine Trifluormethylgruppe,
mit der Maßgabe, daß die Alkylgruppen geradkettig, verzweigt oder cyclisch sein können und die Phenyl- oder Benzylgruppen gegebenenfalls durch $C_1$-$C_6$-Alkylgruppen, $C_1$-$C_6$-Alkoxygruppen, Trifluormethylgruppen, Nitrogruppen oder Halogenatome substituiert sein können,
n 1, 2 oder 3 und
eines der Symbole A, B, C oder D N und die anderen CH bedeuten,
sowie von deren N-Oxiden der heterozyklischen Stickstoffatome und deren Additionssalzen mit pharmazeutisch annehmbaren Säuren und Basen, **dadurch gekennzeichnet**, daß man daß man ein Derivat der Formel II

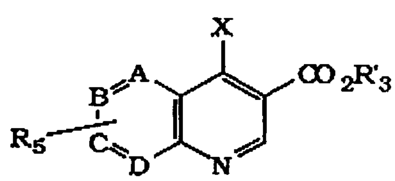

II

in der X ein Halogenatom oder eine Sulfonylgruppe und $R'_3$ eine Alkylgruppe bedeuten und $R_5$, A, B, C und D die bezüglich der Formel I angegebenen Bedeutungen besitzen,
mit einem Amin der Formel III:

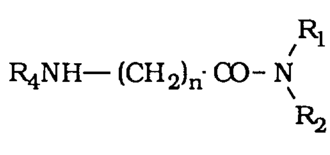

III

in der $R_1$, $R_2$, $R_4$ und n die bezüglich der Formel I angegebenen Bedeutungen besitzen, umsetzt und gegebenenfalls die in dieser Weise erhaltenen Ester (I) zu den entsprechenden Säuren hydrolysiert ($R_3$ = H).

2. Verfahren nach Anspruch 1, **dadurch gekennzeichnet,** daß man Verbindungen der Formel I herstellt, worin A N und B, C und D jeweils CH bedeuten.

3. Verfahren nach einem der Ansprüche 1 und 2, **dadurch gekennzeichnet,** daß man Verbindungen der Formel I herstellt, worin $R_4$ ein Wasserstoffatom und n 1 bedeuten.

4. Verfahren zur Herstellung einer pharmazeutischen Zubereitung enthaltend als Wirkstoff eine Verbindung erhalten nach einem der Ansprüche 1 bis 3, **dadurch gekennzeichnet,** daß man diese Verbindung mit üblicherweise verwendeten Hilfsmitteln kombiniert.